# EUROPEAN PATENT APPLICATION

(11) **EP 4 789 633 A1**
(43) Date of publication of application: **12.08.2026**
(21) Application number: 26157675.5
(22) Date of filing: 10.02.2026
(51) Int. Cl.: A61B 18/12, A61B 18/14

(54) **MONOPOLAR SHIELD CURRENT MONITORING SYSTEMS AND METHODS**

(30) Priority: 10.02.2025 US 202519049408
(71) Applicant: Cilag GmbH International, 6300 Zug (CH)
(72) Inventor: SAMUEL, Jonathan, Cincinnati, 45242 (US); BONILLA, Osvaldo A., Cincinnati, 45242 (US)
(74) Representative: Carpmaels & Ransford LLP

(57) **Abstract**

A surgical system is disclosed including a surgical instrument and a controller in operable communication with the surgical instrument and an energy generator. The surgical instrument comprises a shaft, an electrode extending within the shaft, and a shield capacitively coupled to the electrode. The controller is operable to provide a voltage to the electrode from the energy generator, receive a shield current from the shield based on providing the voltage to the electrode, and determine a status of the surgical instrument based on the received shield current.

## Description

### BACKGROUND

The present disclosure relates to surgical systems and, more particularly, to energy devices used during surgical procedures and systems for monitoring fault conditions thereof.

During a surgical procedure, an energy device can be used to provide energy to the tissue of a patient. A fault condition may cause the energy device to not operate as intended, or may inadvertently cause energy to be provided to the patient at an unintended location. Accordingly, systems and methods for detecting fault conditions of an energy device are desired.

### BRIEF DESCRIPTION OF THE DRAWINGS

The following figures are included to illustrate certain aspects of the present disclosure, and should not be viewed as exclusive embodiments. The subject matter disclosed is capable of considerable modifications, alterations, combinations, and equivalents in form and function, without departing from the scope of this disclosure.
FIG. 1 is a block diagram of a computer-implemented interactive surgical system, in accordance with at least one aspect of the present disclosure.
FIG. 2 is a diagram of various modules, including an energy module and an evacuator module, and other components that are combinable to customize modular energy systems, in accordance with at least one aspect of the present disclosure.
FIG. 3 is the energy module of FIG. 2 and various surgical instruments usable therewith, in accordance with at least one aspect of the present disclosure.
FIG. 4A is a first illustrative modular energy system configuration including a header module and a display screen that renders a graphical user interface (GUI) for relaying information regarding modules connected to the header module, in accordance with at least one aspect of the present disclosure.
FIG. 4B is an isometric view of the modular energy system shown in FIG. 4A mounted to a cart, in accordance with at least one aspect of the present disclosure.
FIG. 5 is a second illustrative modular energy system configuration including a header module, a display screen, two energy modules, and an evacuator module connected together and mounted to a cart, in accordance with at least one aspect of the present disclosure.
FIG. 6 is a block diagram of a hub configuration of a modular energy system, in accordance with at least one aspect of the present disclosure.
FIG. 7 is an electrosurgical instrument, in accordance with at least one aspect of the present disclosure.
FIG. 8 is a graph providing power curves for operating modes of the electrosurgical instrument of FIG. 7, in accordance with at least one aspect of the present disclosure.

### DETAILED DESCRIPTION

The present disclosure relates to surgical systems and, more particularly, energy devices used during surgical procedures and systems for monitoring fault conditions thereof.

Energy devices use energy to affect (treat) tissue. In an energy device, the energy is supplied by a generator. Energy devices include tissue-contacting electrodes, such as one or more radio frequency (RF) electrodes, and the generator is configured to generate oscillating electric currents to energize the electrodes. The generator may be configured to detect fault conditions of the energy device, which may cause the energy device to not operate as intended.

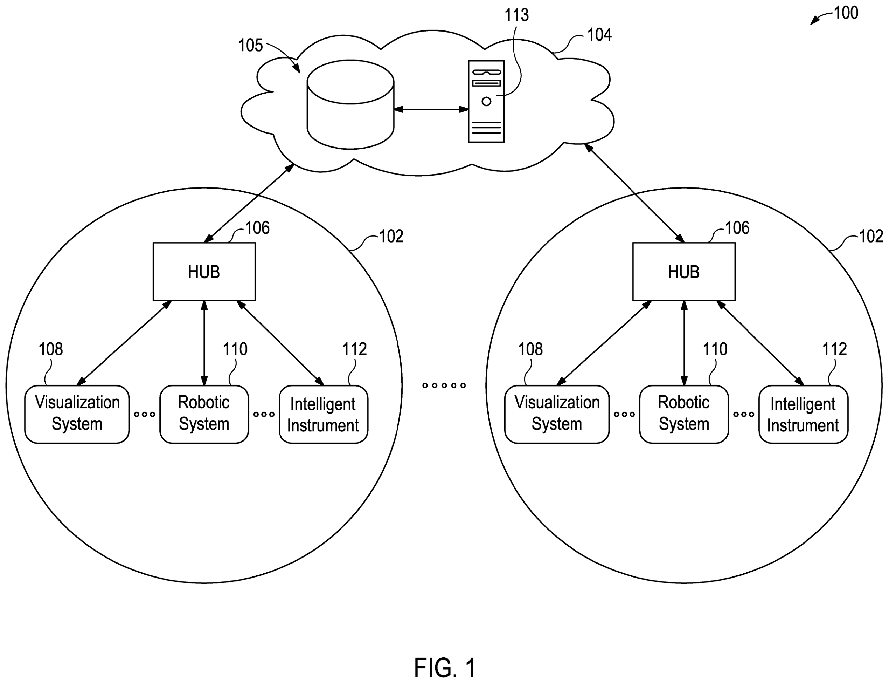

FIG. 1 is a block diagram of a computer-implemented interactive surgical system 100 (hereinafter "the surgical system 100") that may be used in accordance with at least one aspect of the present disclosure. The surgical system 100 includes one or more sub-surgical systems 102 and a cloud-based system (e.g., the cloud 104) that may include a remote server 113 in communication with a storage device 105. Each sub-surgical system 102 includes at least one surgical hub 106 in communication with the cloud 104 that may include a remote server 113.

In one example, as illustrated in FIG. 1, each sub-surgical system 102 includes a visualization system 108, a robotic system 110, and a handheld intelligent surgical instrument 112, which are configured to communicate with one another and/or the hub 106. In some aspects, each sub-surgical system 102 may include an M number of hubs 106, an N number of visualization systems 108, an O number of robotic systems 110, and a P number of handheld intelligent surgical instruments 112, where M, N, O, and P are integers greater than or equal to one. The surgical system 100 is described in more detail in U.S. Patent No. 11,666,368, entitled "METHOD FOR CONSTRUCTING AND USING A MODULAR SURGICAL ENERGY SYSTEM WITH MULTIPLE DEVICES", issued June 6, 2023, and is hereby incorporated by reference in its entirety herein.

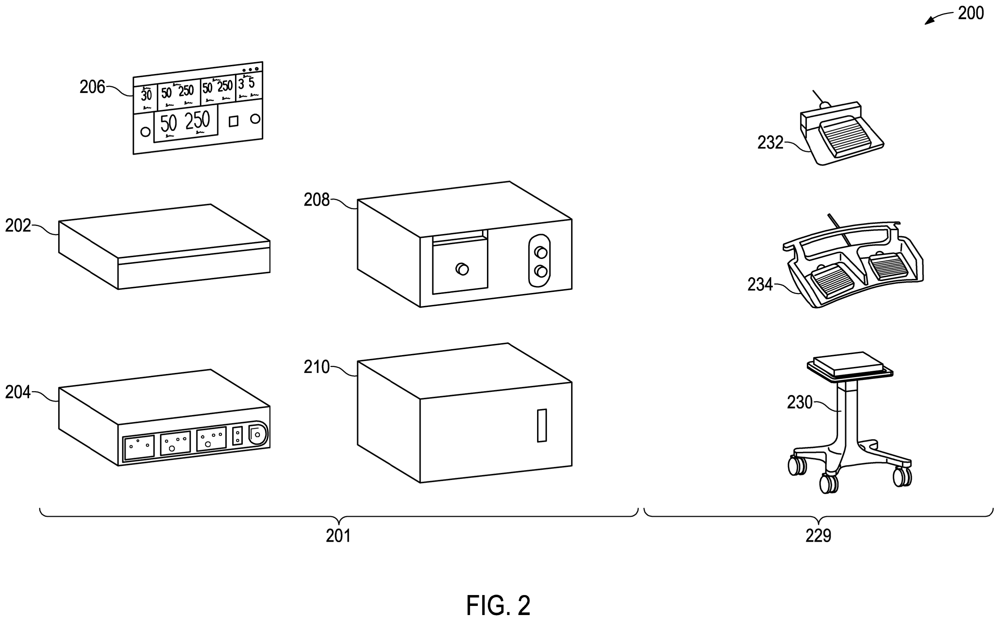

Referring now to FIG. 2, an example surgical hub 106 (FIG. 1) can be embodied as a modular energy system 200 that can include a variety of different modules 201 that are connectable together in a stacked configuration. In one aspect, the modules 201 can be both physically and communicably coupled together when stacked or otherwise connected together into a singular assembly. Further, the modules 201 can be interchangeably connectable together in different combinations or arrangements. In one aspect, each of the modules 201 can include a consistent or universal array of connectors disposed along their upper and lower surfaces, thereby allowing any module 201 to be connected to another module 201 in any arrangement (except that, in some aspects, a particular module type, such as the header module 202, can be configured to serve as the uppermost module within the stack, for example). In an alternative aspect, the modular energy system 200 can include a housing that is configured to receive and retain the modules 201. The modular energy system 200 can also include a variety of different components or accessories that are also connectable to or otherwise associatable with the modules 201.

The modular energy system 200 can be assembled from a variety of different modules 201, some examples of which are illustrated in FIG. 2. Each of the different types of modules 201 can provide different functionality, thereby allowing the modular energy system 200 to be assembled into different configurations to customize the functions and capabilities of the modular energy system 200 (e.g., by customizing the modules 201 that are included in each modular energy system 200). The modules 201 of the modular energy system 200 can include, for example, a header module 202 (which can include a display screen 206), an energy module 204, an evacuator module 208, and a visualization module 210.

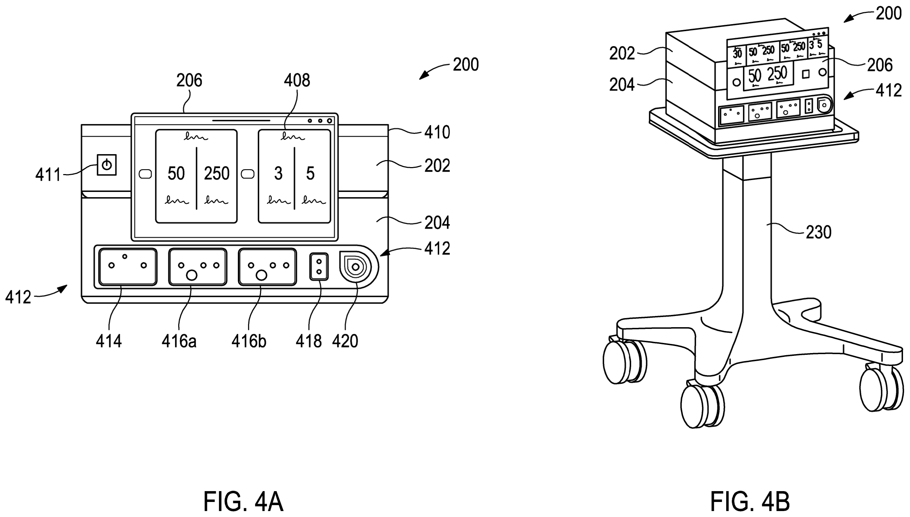

In the depicted aspect, the header module 202 is configured to serve as the top or uppermost module within the modular energy system stack and can thus lack connectors along its top surface. In another aspect, the header module 202 can be configured to be positioned at the bottom or the lowermost module within the modular energy system stack (*i.e*., a "footer" module) and can thus lack connectors along its bottom surface. In yet another aspect, the header module 202 can be configured to be positioned at an intermediate position within the modular energy system stack and can thus include connectors along both its bottom and top surfaces. The header module 202 can be configured to control the system-wide settings of each module 201 and component connected thereto through physical controls 411 (FIG. 4A) thereon and/or a graphical user interface (GUI) 408 (FIG. 4A) rendered on the display screen 206. Such settings could include the activation of the modular energy system 200, the volume of alerts, the footswitch settings, the settings icons, the appearance or configuration of the user interface, the surgeon profile logged into the modular energy system 200, and/or the type of surgical procedure being performed. The header module 202 can also be configured to provide communications, processing, and/or power for the modules 201 that are connected to the header module 202.

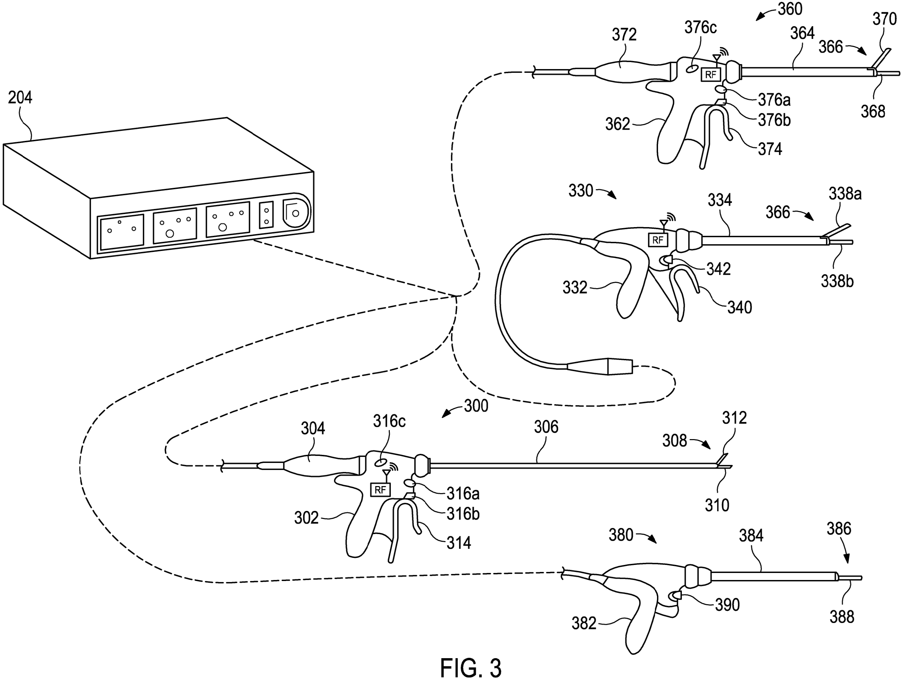

The energy module 204, alternately referred to as a generator module, can be configured to generate one or multiple energy modalities for driving electrosurgical and/or ultrasonic surgical instruments connected thereto. For example, referring to FIG. 3, the generator 204 is configured to drive multiple surgical instruments 300, 330, 360, 380. The first surgical instrument is an ultrasonic surgical instrument 300 and comprises a handpiece 302 (HP), an ultrasonic transducer 304, a shaft 306, and an end effector 308. The end effector 308 comprises an ultrasonic blade 310 acoustically coupled to the ultrasonic transducer 304 and a clamp arm 312. The handpiece 302 comprises a trigger 314 to operate the clamp arm 312 and a combination of toggle buttons 316a, 316b, 316c to energize and drive the ultrasonic blade 310 or other function. The toggle buttons 316a-c can be configured to energize the ultrasonic transducer 304 with the generator 204.

The generator 204 is also configured to drive the second surgical instrument 330, which is an RF electrosurgical instrument and comprises a handpiece 332 (HP), a shaft 334, and an end effector 336. The end effector 336 comprises clamp arms 338a, 338b that are configured to grasp patient tissue therebetween. In some embodiments, both clamp arms 338a, 338b may include an electrode that may be energized by a bipolar energy source within the energy module 204 to apply bipolar energy to patient tissue grasped between the clamp arms 338a, 338b. In other embodiments, only one of the clamp arms 338a, 338b includes an electrode that may be energized by a monopolar energy source within the energy module 204 to apply monopolar energy to patient tissue grasped between the clamp arms 338a, 338b.. The handpiece 332 includes a trigger 340 manually actuatable to operate (position) the clamp arms 338a,b, and an energy button 342 to actuate an energy switch to energize the electrode(s) in the end effector 336. The electrode(s) may also be energized by the foot switches 232, 234 (FIG. 2), discussed in more detail elsewhere herein.

The generator 204 is also configured to drive the third surgical instrument 360, which is a multifunction surgical instrument 360 and comprises a handpiece 362 (HP), a shaft 364, and an end effector 366. The end effector 366 comprises an ultrasonic blade 368 and a clamp arm 370. The ultrasonic blade 368 is acoustically coupled to an ultrasonic transducer 372. The handpiece 362 includes a trigger 374 to operate the clamp arm 370, and a combination of toggle buttons 376a, 376b, 376c to energize and drive the ultrasonic blade 368 or other function. The toggle buttons 376a-c can be configured to energize the ultrasonic transducer 372 with the generator 204 and energize the ultrasonic blade 368 with a bipolar energy source also contained within the generator 204.

The generator 204 is also configured to drive the fourth surgical instrument 380, which is a monopolar surgical instrument 380 that comprises a handpiece 382 (HP), a shaft 384, and an end effector 386. The end effector 386 comprises an electrode 388. The handpiece 382 includes an energy button 390 to energize the electrode 388 to deliver monopolar energy to tissue. Further aspects of the surgical instruments are described in U.S. Patent No. 10,624,691, entitled "TECHNIQUES FOR OPERATING GENERATOR FOR DIGITALLY GENERATING ELECTRICAL SIGNAL WAVEFORMS AND SURGICAL INSTRUMENTS", issued April 21, 2020, which is herein incorporated by reference in its entirety herein.

The evacuator module 208 (FIG. 2) can be configured to evacuate smoke, fluid, and/or particulates generated by the application of therapeutic energy to the tissue by one or more of the surgical instruments 300, 330, 360, 380. Example evacuator modules are described in more detail elsewhere herein, as well as in U.S. Patent No. 11,602,393, entitled "SURGICAL EVACUATION SENSING AND GENERATOR CONTROL", issued March 14, 2023, which is hereby incorporated by reference in its entirety herein.

The visualization module 210 (FIG. 2) can be configured to interface with visualization devices (i.e., scopes) and accordingly provide increased visualization capabilities. Example visualization modules and systems are described in more detail in U.S. Patent No. 11,284,963, entitled "METHOD OF USING IMAGING DEVICES IN SURGERY", issued March 29, 2022, which is hereby incorporated by reference in its entirety herein.

Referring again to FIG. 2, the modular energy system 200 can further include a variety of accessories 229 that are connectable to the modules 201 for controlling the functions thereof or that are otherwise configured to work in conjunction with the modular energy system 200. The accessories 229 can include, for example, a single-pedal footswitch 232, a dual-pedal footswitch 234, and a cart 230 for supporting the modular energy system 200 thereon. The footswitches 232, 234 can be configured to control the activation or function of particular energy modalities output by the energy module 204, for example.

By utilizing modular components, the depicted modular energy system 200 provides a surgical platform that grows with the availability of technology and is customizable to the needs of the facility and/or surgeons. Further, the modular energy system 200 supports combo devices (e.g., dual electrosurgical and ultrasonic energy generators) and supports software-driven algorithms for customized tissue effects. Still further, the surgical system architecture reduces the capital footprint by combining multiple technologies critical for surgery into a single system.

The various modular components utilizable in connection with the modular energy system 200 can include monopolar energy generators, bipolar energy generators, dual electrosurgical/ultrasonic energy generators, display screens, and various other modules and/or other components described elsewhere herein.

Referring now to FIG. 4A, the header module 202 can, in some aspects, include the display screen 206 that renders a GUI 408 for relaying information regarding the modules 201 (FIG. 2) connected to the header module 202. In some aspects, the GUI 408 of the display screen 206 can provide a consolidated point of control of all of the modules 201 making up the particular configuration of the modular energy system 200. In alternative aspects, the header module 202 can lack the display screen 206, or the display screen 206 can be detachably connected (removably attachable) to a housing 410 of the header module 202. In such aspects, the header module 202 can be communicably couplable to an external system that is configured to display the information generated by the modules 201 of the modular energy system 200. For example, in robotic surgical applications, the modular energy system 200 can be communicably couplable to a robotic cart or robotic control console, which is configured to display the information generated by the modular energy system 200 to the operator of the robotic surgical system. As another example, the modular energy system 200 can be communicably couplable to a mobile display that can be carried or secured to a surgical staff member for viewing thereby. In aspects utilizing a user interface that is separate from or otherwise distinct from the modular energy system 200, the user interface can be wirelessly connectable with the modular energy system 200 as a whole or one or more modules 201 thereof such that the user interface can display information from the connected modules 20001 thereon.

Referring still to FIG. 4A, the energy module 204 can include a port assembly 412 including (providing) a number of different ports configured to deliver different energy modalities to corresponding surgical instruments (e.g., surgical instruments 300, 330, 360, 380 of FIG. 3, for example) that are connectable thereto. In the particular aspect illustrated in FIGS. 4A, the port assembly 412 includes a bipolar port 414, a first monopolar port 416a, a second monopolar port 416b, a neutral electrode port 418 (to which a monopolar return pad is connectable), and a combination energy port 420. However, this particular combination of ports is simply provided for illustrative purposes and alternative combinations of ports and/or energy modalities may be possible for the port assembly 412.

As noted above, the modular energy system 200 can be assembled into different configurations. Further, the different configurations of the modular energy system 200 can also be utilizable for different surgical procedure types and/or different tasks. For example, FIGS. 4A and 4B illustrate a first illustrative configuration of the modular energy system 200 including the header module 202 (including the display screen 206) and the energy module 204 connected together. Such a configuration can be suitable for laparoscopic and open surgical procedures, for example. As shown in FIG. 4B, the modular energy system 200 can be positioned on a cart 230 enabling the modular energy system 200 to be easily moved (wheeled) around the operating room, for example.

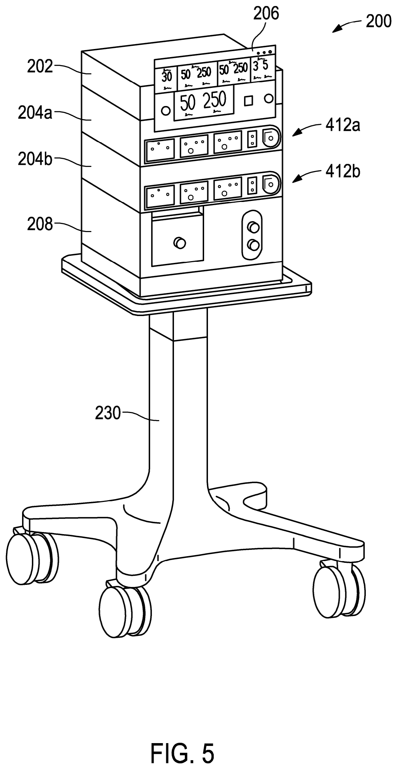

FIG. 5 illustrates a second illustrative configuration of the modular energy system 200 including the header module 202 (including the display screen 206), a first energy module 204a, a second energy module 204b, and the evacuator module 208 connected together and positioned on the cart 230. In such a configuration, the evacuator module 208 can evacuate smoke, fluid, and/or particulates generated by surgical instruments powered by the energy modules 204a,b.

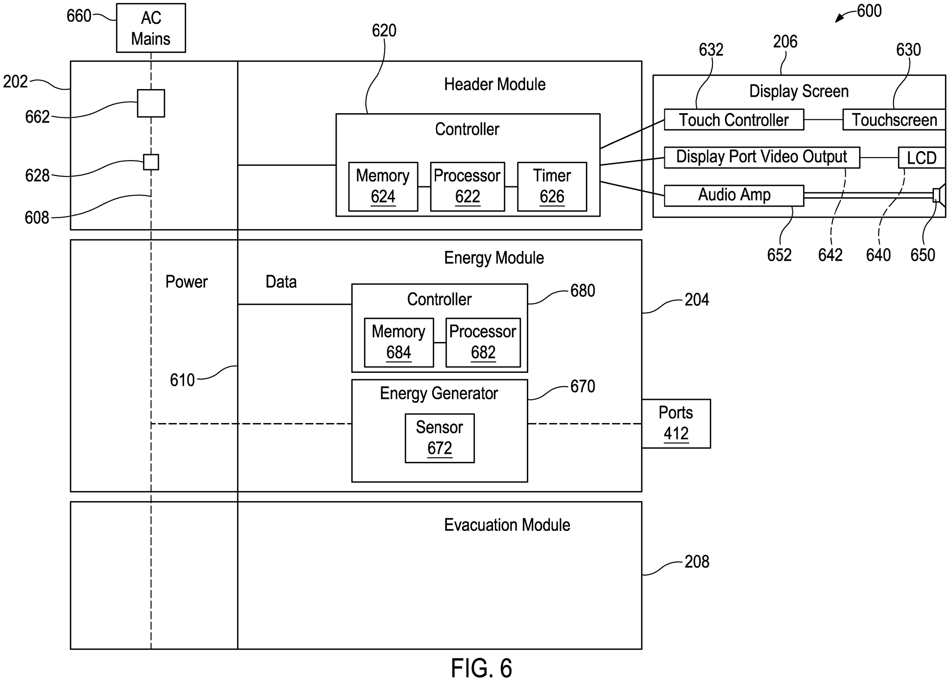

FIG. 6 is a block diagram of an example modular energy system 600, in accordance with at least one aspect of the present disclosure. As illustrated, the modular energy system 600 includes the header module 202 (including the display screen 206), the energy module 204 stacked under and coupled to the header module 202, and the evacuator module 208 stacked under and coupled to the energy module 204.

The header module 202 is configured to monitor, control, energize, and provide feedback concerning operation of the modules within the modular energy system 600, such as the energy module 204 and the evacuator module 208. As illustrated, the header module 202 includes a controller 620 that comprises a processor 622 and a memory 624 storing computer readable instructions executable by the processor 622 to carry out functions and operations of the header module 602. Examples of the memory 624 include, but are not limited to, random access memory (RAM), read-only memory (ROM), computer chips, optical discs (e.g., compact discs (CDs), digital video discs (DVDs), etc.), magnetic disks (e.g., hard disk drives (HDDs), floppy disks, ZIP^{®} disks, etc.), magnetic tape, and solid state storage devices (e.g., memory cards, "flash" media, etc.). As used herein, the term "computer readable medium" refers to any device or system for storing and providing information (e.g., data and instructions) to the processor 622. Examples of computer readable media include, but are not limited to, optical discs, magnetic disks, magnetic tape, solid-state media, and servers for streaming media over networks.

Based on instructions stored in the memory 624, the processor 622 may be configured to control power and data transmissions between the header module 202, the energy module 204, and the evacuator module 208 through a power interface 608 and a data interface 610. For example, the header module 202 can transmit various commands to the energy module 204 and evacuator module 208 (through the energy module 204) via the data interface 610. Such commands can be based on user inputs received at the display screen 206 or inputs received by the controller 620 from various sensors communicably coupled to the modular energy system 600, as discussed elsewhere herein.

As a further example, power may be transmitted to the energy module 204 and the evacuator module 208 (through the energy module 204) from the header module 202 via the power interface 608. The header module 202 may receive power from an external power source 660 (referred to herein as "AC Mains"), such as a wall outlet, for example. The header module 202 may include an AC/DC converter 662 which receives the AC power from the AC Mains 660 and converts the AC power to DC power. The controller 620 may then distribute the DC power to the energy module 204 and the evacuator module 208. The controller 620 may further include a timer 626 for measuring elapsed time. The header module 202 may include a sensor 628, such as a current sensor and/or a power sensor, for example, in operable communication with the controller 620 for measuring current and power along the power interface 608.

As shown in FIG. 6, the energy module 204 may include a controller 680 that comprises a processor 682 and a memory 684 storing computer readable instructions executable by the processor 682 to carry out functions and operations of the energy module 204. The processor 682 and a memory 684 may be similar to processor 622 and memory 624, respectively. The controller 680 may receive power from the AC/DC converter 662 along the power interface 608 and may be in operable communication with controller 620 via the data interface 610.

The energy module 204 may further include an energy generator 670. The energy generator 670 may receive power from the AC/DC converter 662 along the power interface 608 and may be in operable communication with controller 680, such as via a wired or wireless connection. The energy generator 670 may be operable to provide therapeutic energy to one or more surgical instruments, such as the surgical instruments 300, 330, 360, 380, via the port assembly 412, such as via the bipolar port 414 (FIG. 4), the first or second monopolar ports 416a, 416b (FIG. 4), or the combination energy port 420 (FIG. 4), for example. For instance, the energy generator 670 may be energized with DC power provided thereto from the AC/DC converter 662 along the power interface 608. The controller 680 may then receive an input, such as from the controller 620, and based on the input, the controller 680 may direct the energy generator 670 to provide therapeutic energy to one or more surgical instruments coupled to the energy module 204 at the port assembly 412. The energy generator 670 may include a sensor 672, such as a current sensor and/or a power sensor in operable communication with the controller 680 for measuring current and/or power provided by the energy generator 670. The sensor 672 may also comprise an impedance sensor for measuring the impedance of tissue grasped by one of the surgical instruments.

As shown in FIG. 6, the display screen 206 includes a touchscreen 630 coupled to a touch controller 632. The touch controller 632 is coupled to the controller 620 to read inputs, such as user inputs, from the touchscreen 630. The controller 620 drives an LCD display 640 through a display/port video output signal 642. The controller 620 is further coupled to an audio amplifier 652 to drive one or more speakers 650.

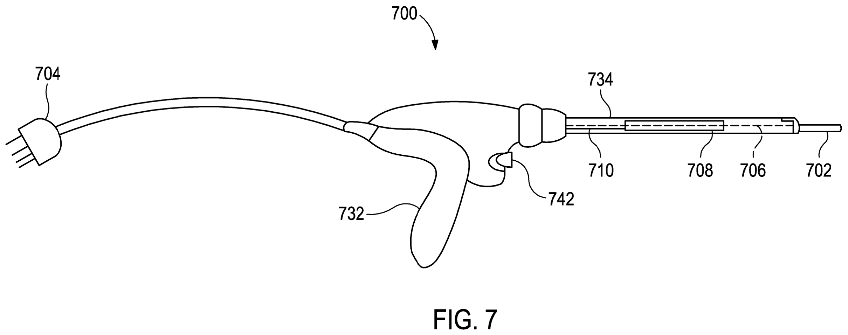

FIG. 7 is a schematic side view of an example monopolar surgical instrument 700 that may incorporate one or more aspects of the present disclosure. The monopolar surgical instrument 700 may be the same as or similar to the monopolar instrument 380 (FIG. 3).

As illustrated, the monopolar surgical instrument 700 may comprise a handpiece or "housing" 732, a shaft 734 extending from the housing 732, and an end effector 736 provided at a distal end of the shaft 734. In the illustrated application, the end effector 736 includes an electrode 702.

The housing 732 may include an energy button 742 actuatable by a user to energize the electrode 702, thereby providing monopolar energy to the tissue of a patient, as discussed in more detail below. Alternatively, or in combination therewith, the electrode 702 may be energized based on a user actuating one of the foot switches 232, 234 (FIG. 2).

The monopolar surgical instrument 700 may further include a plug 704 extending from the housing 732 and which may be couplable to one of the monopolar ports 416a,b (FIG. 4A) of the energy module 204 (FIG. 4A) to electrically couple the monopolar surgical instrument 700 to the energy module 204. The monopolar surgical instrument 700 may further include an electrode conductor (wire) 706 that extends from the plug 704 to the electrode 702 and which functions to electrically couple the electrode 702 to the energy module 204 when the plug 704 is coupled to one of the monopolar ports 416a,b.

Referring now to FIGS. 6 and 7, during a surgical procedure, a user (clinician) may desire to provide monopolar energy to the tissue of a patient. Accordingly, the user may couple the plug 704 of the monopolar surgical instrument 700 to one of the monopolar ports of the energy module 204, such as the monopolar port 416a (FIG. 4A) of the modular energy system 600. The user may also plug (couple) a monopolar return pad (not shown) to the neutral electrode port 418 (FIG. 4A) of the energy module 204 of the modular energy system 600 and couple the monopolar return pad to the patient, thereby providing a return path for the monopolar energy provided by the monopolar surgical instrument 700.

The user may also provide one or more inputs to the modular energy system 600. For instance, the user may provide an input to the touchscreen 630 regarding a desired operating mode (state) of the monopolar surgical instrument 700. The operating mode may include a first operating mode in which the controller 680 sets a first power curve, a second operating mode in which the controller 680 sets a second power curve different (greater) than the first power curve, or a third operating mode in which the controller 680 sets a third power curve different (greater) than the second power curve. The operating modes and associated power curves may be stored in the memory 684.

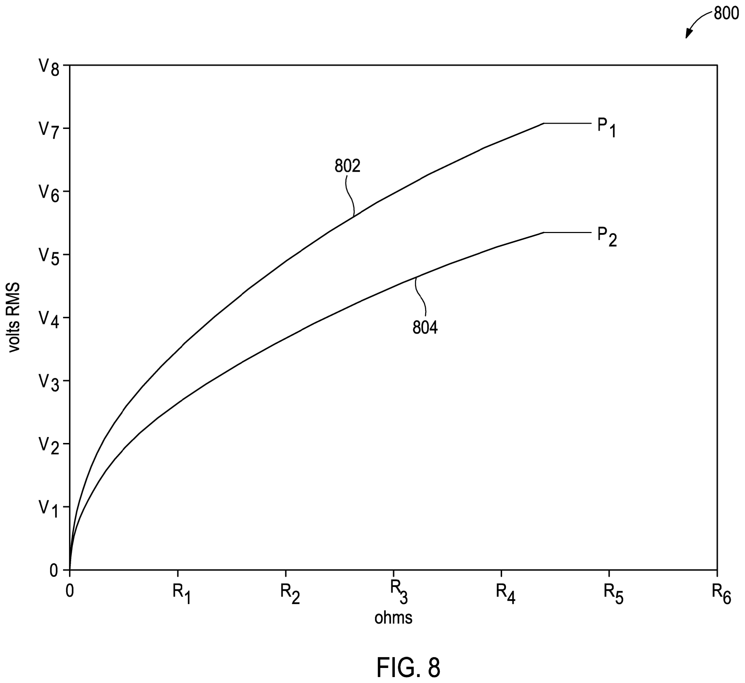

FIG. 8 depicts a graph 800 that may be stored in the memory 684 and that illustrates a first power curve (P₁) 802 corresponding to a first operating mode of the monopolar surgical instrument 700 and a second power curve (P₂) 804 corresponding to second operating mode of the monopolar surgical instrument 700, wherein the wattage of the first power curve P₁ (*e.g*. 35W) is greater than the wattage of the second power curve P₂ (*e.g.* 20W). The x-axis 806 of the graph 800 may reflect measured tissue impedance, which may be in ohms, and the y-axis 808 of the graph 800 may be output voltage (V_{RMS}), which may be volts. While the graph 800 only provides two power curves 802, 804, the graph 800 may include additional power curves corresponding to the number of desired operating modes of the monopolar surgical instrument 700. The graph 800 and associated values of the power curves 802, 804 may also be stored in the memory 684 in the form of a look-up table.

Once the user has selected a desired operating mode of the monopolar surgical instrument 700, the user may manipulate the monopolar surgical instrument 700 such that the electrode is situated against (engaged with) tissue to be energized. Once satisfied with the position of the electrode 702, the user may actuate the energy button 742 (or one of the footswitches 232, 234), thereby causing the controller 680 of the energy module 204 to control the energy generator 670 and thereby provide a first or "sub-therapeutic" voltage to the electrode 702. The sub-therapeutic voltage may be a voltage sufficiently low enough such that no, or at least substantially no, tissue effect is applied to the tissue. Rather, the sub-therapeutic voltage may be a voltage that is sufficient to allow the controller 680 to measure (determine) the impedance of the tissue positioned against the electrode 702, such as via the sensor 672. The sub-therapeutic voltage may be stored in the memory 684.

Once the impedance of the tissue is determined, the controller 680 may adjust the first voltage to a second or "therapeutic" voltage. For instance, the controller 680 may retrieve, from the memory 684, the graph 800 and determine the second voltage (y-axis 808) based on the measured impedance (x-axis 806) and the power curve associated with the selected operating mode. As an example, based on a user selecting the first operating mode of the monopolar surgical instrument 700 with the first power curve P₁ 802 and the controller 680 measuring a tissue impedance of R₃, the controller 680 may be configured to set the energy generator 670 to output a second voltage of V₆.

Once the second voltage is set, the controller 680 may then control the energy generator 670 to provide the second voltage to the electrode 702 via the electrode conductor 706, thereby causing a tissue effect (e.g. tissue coagulation).

During the surgical procedure, the controller 680 may continuously or periodically measure the impedance of the tissue as the energy generator 670 applies energy to the tissue via the electrode 702. If the controller 680 detects a change in impedance, the controller 680 may adjust the second voltage according to the new measured impedance and the set power curve. Accordingly, the controller 680 may continuously, or periodically, adjust the second voltage output by the energy generator 670 to the electrode 702 based on the controller 680 detecting a change in impedance of the tissue.

In some instances, the shaft 734 of the monopolar surgical instrument 700 may be constructed (made) of metal or any other suitable electrically conductive material, such as stainless steel. Due to the metallic/conductive construction of the shaft 734, when the therapeutic energy is provided to the electrode 702 via the electrode conductor 706, as discussed above, the electrode conductor 706 may capacitively couple with the metallic shaft 734, thereby causing current to be induced in the shaft 734. This induced current may cause the shaft 734 to inadvertently burn tissue that comes into contact therewith.

To avoid inadvertent tissue burn from the shaft 734, the monopolar surgical instrument 700 may further include a shield 708 and a shield conductor

(wire) 710. The shield 708 may be positioned (concentrically) about at least a portion of the electrode conductor 706, between (interposing) the electrode conductor 706 and the metallic shaft 734. The shield conductor 710 may extend from the shield 708, within the shaft 734 and alongside the electrode conductor 706, to the plug 704 such that, when the plug 704 is coupled to one of the monopolar ports of the energy module 204, such as the monopolar port 416a, the shield conductor 710 may also be electrically coupled to the energy module 204. Accordingly, when energy is provided to the electrode 702 via the electrode conductor 706, the electrode conductor 706 may capacitively couple with the shield 708, in lieu of the shaft 734, thereby causing current induced in the shield 708 to be drained (conveyed) to the energy module 204 via the shield conductor 710. The shield 708 may be made of any suitable electrically conductive material, such as a metal.

Based on the plug 704 being coupled to the energy module 204, as described above, the controller 680 of the energy module 204 may monitor (sense) the current drained from the shield 708 via the shield conductor 710 (hereinafter referred to as "shield current"), such as with the sensor 672. By monitoring the shield current, the controller 680 may determine a status or condition of the monopolar surgical instrument 700, such as a status or condition of the shield 708, to determine (detect) any abnormalities therewith.

More specifically, the controller 680 may expect to receive a particular shield current (hereinafter referred to as "expected shield current") for a shield 708 that is intact or "undamaged" and that is properly positioned within the shaft 734 while providing energy to the electrode 702, as discussed above. The expected shield current may be a function of the voltage applied to the electrode 702 (*e.g.* the therapeutic voltage), the selected operating mode of the monopolar surgical instrument 700, the capacitance between the electrode conductor 706 and the shield 708, or the capacitance between the electrode conductor 706 and the shield conductor 710, or combinations thereof.

The expected shield current for the various combinations of the aforementioned parameters may be predetermined, stored in the memory 684, and retrievable by the controller 680. Alternatively, the controller 680 may continuously, or periodically, calculate the expected shield current based on the set output voltage, the operating mode of the monopolar surgical instrument 700, and the foregoing capacitance. The foregoing capacitance may also be stored in the memory 684 and retrieved by the controller 680. Alternatively, or in combination therewith, the foregoing capacitance may be provided to the controller 680, such as by the user providing the values via the touchscreen 630 or by a user scanning a barcode of a packaging associated with the monopolar surgical instrument 700. Alternatively, or in combination therewith, the foregoing capacitance may be determined by the controller by measuring current from the shield conductor 710 when a "new" monopolar surgical instrument 700 is initially connected to the generator 204, the assumption being that the "new" monopolar surgical instrument 700 includes an undamaged shield 708.

As mentioned herein above, the voltage provided to the electrode 702 by the energy generator 670 may be continuously, or periodically, adjusted due to the changing impedance of the tissue to which the electrode 702 is providing energy. Accordingly, as the expected shield current is a function of the voltage provided to the electrode 702, the expected shield current expected to be received by the controller 680 may similarly be continuously, or periodically, adjusted.

Based on the energy generator 670 providing the therapeutic voltage to the electrode conductor 706 and the electrode 702, as discussed above, the controller 680 may measure (sense) an actual shield current from the shield conductor 710, such as via the sensor 672. The controller 680 may compare the actual shield current to the expected shield current to determine the status or condition of the monopolar surgical instrument 700. The controller 680 may detect that the actual shield current is a threshold amount less than or greater than the expected shield current. If the controller 680 detects that the actual shield current is a threshold amount greater than the expected shield current, the controller 680 may conclude that the electrode conductor 706 is in contact with another portion of the monopolar surgical instrument 700, such as the shaft 734, the shield 708, or the shield conductor 710, thereby causing the current from the electrode conductor 706 to short circuit (*e.g*. a "short circuit" or "low impedance" condition). If the controller 680 detects that the actual shield current is a threshold amount less than the expected shield current, the controller 680 may conclude that the shield 708 is damaged (partially or completely) or that the shield conductor 710 has become disconnected from the shield 708, which may mean that the shield 708 is not properly performing the function of preventing the electrode conductor 706 from capacitively coupling to the shaft 734 (*e.g.* an "open circuit" or "high impedance" condition). If the controller 680 detects that the actual shield current is within a threshold range from the expected shield current (*e.g*. not above or below the threshold amounts), the controller 680 may conclude that the shield 708 is properly functioning. The threshold amounts and range may be stored in the memory 684 and may be retrieved by the controller 680.

The arrangement of the foregoing monopolar surgical instrument 700 enables to a user to detect the status of the monopolar surgical instrument 700 at any time that the energy generator 670 outputs energy to the monopolar surgical instrument 700, such as during, prior to, or after a surgical procedure. For instance, during a surgical procedure, a user may desire to know if the shield 708 is properly functioning prior to positioning the electrode 702 against tissue. Accordingly, the user may energize the electrode 702 in the air (*i.e.* spaced away from the patient and tissue). The controller 680 may measure the actual shield current and compare it to an expected shield current to determine if the shield 708 is properly functioning, as described herein above. The controller 680 may perform an action based on the detection of a fault condition (*e.g*. low or high impedance condition). For instance, the controller 680 may control the energy generator 670 to cease providing energy to the monopolar surgical instrument 700 and/or may provide an alert to a user, such as a visual alert via the LCD 640 and/or an audible alert via the speaker 650.

Embodiments disclosed herein include:
A. A surgical system comprising and surgical instrument and a controller in operable communication with the surgical instrument and an energy generator. The surgical instrument comprises a shaft, a conductor extending within the shaft, and a shield capacitively coupled to the conductor. The controller is operable to provide a voltage to the conductor from the energy generator, receive a shield current from the shield based on providing the voltage to the conductor, and determine a status of the surgical instrument based on the received shield current.
B. A surgical system comprising a surgical instrument and a controller in operable communication with the surgical instrument and an energy generator. The surgical instrument comprises a shaft, an electrode arranged at an end of the shaft and energizable to provide energy to tissue, a conductor extending within the shaft and electrically coupled to the electrode, and a shield capacitively coupled to the conductor. The controller is operable to receive an input indicative of an operating mode of the surgical instrument, provide a voltage to the conductor and the electrode from the energy generator in the operating mode, determine an expected shield current based on the operating mode of the surgical instrument and the voltage, receive an actual shield current from the shield based on providing the voltage, and determine a condition of the surgical instrument based on the expected shield current and the actual shield current.
C. A non-transitory computer readable medium storing instructions that, when executed by a processor, cause the processor to receive an input indicative of an operating mode of a surgical instrument, control an energy generator to provide a voltage to a conductor of the surgical instrument, the conductor being capacitively coupled to a shield of the surgical instrument, determine an expected shield current based on the operating mode of the surgical instrument and the voltage, receive an actual shield current from the shield based on providing the voltage, compare the expected shield current and the actual shield current, and detect an abnormality in the surgical instrument based on the comparison.

Each of embodiments A-C may have one or more of the following additional elements in any combination: Element 1: wherein the controller is further operable to determine an expected shield current based on the provided voltage. Element 2: wherein the controller is further operable to receive an input indicative of a selected operating mode of the surgical instrument, and wherein the expected shield current is further based on the selected operating mode. Element 3: wherein the controller is further operable to compare the shield current to the expected shield current and determine the status of the surgical instrument based on the comparison. Element 4: wherein the status of the surgical instrument comprises a low impedance condition based on the received shield current being a threshold amount greater than the expected shield current. Element 5: wherein the status of the surgical instrument comprises a high impedance condition based on the received shield current being a threshold amount less than the expected shield current. Element 6: wherein the status of the surgical instrument comprises a status of the shield. Element 7: wherein the shaft is comprised of a conductive material. Element 8: wherein the voltage is a second voltage and the surgical instrument further comprises an electrode electrically coupled to the conductor and operable to provide energy to tissue, wherein the controller is further operable to provide a first voltage to the tissue from the energy generator via the conductor and the electrode, determine an impedance of the tissue based on providing the first voltage, and determine the second voltage to provide to the conductor based on the determined impedance. Element 9: wherein determining the condition of the surgical instrument comprises comparing the actual shield current to the expected shield current. Element 10: wherein the condition of the surgical instrument comprises a low impedance condition based on the actual shield current being a threshold amount greater than the expected shield current. Element 11: wherein the condition of the surgical instrument comprises a high impedance condition based on the actual shield current being a threshold amount less than the expected shield current. Element 12: wherein the condition of the surgical instrument comprises a condition of the shield. Element 13: wherein the shaft is comprised of a conductive material. Element 14: wherein the voltage is a second voltage and the controller is further operable to provide a first voltage to the conductor and the electrode, determine an impedance of the tissue based on providing the first voltage, and determine the second voltage to provide to the conductor and the electrode based on the determined impedance. Element 15: wherein the abnormality comprises a high impedance condition of the surgical instrument based on the actual shield current being a threshold amount below the expected shield current. Element 16: wherein the abnormality comprises a low impedance condition of the surgical instrument based on the actual shield current being a threshold amount above the expected shield current. Element 17: wherein the abnormality of the surgical instrument comprises an abnormality in the shield.

By way of non-limiting example, exemplary combinations applicable to A, B, and C include: Element 1 with Element 2; Element 1 with Element 3; Element 1 with Elements 3 and 4; Element 1 with Elements 3 and 5; Element 1 with one or more of Elements 2-8; Element 6 with one or more of Elements 1-5, 7, and 8, Element 7 with one or more of Elements 1-6 and 8; Element 8 with one or more of Elements 1-7; Element 9 with Element 10; Element 9 with Element 11; Element 9 with one or more of Elements 10-14; Element 12 with one or more of Elements 9-11, 13, and 14; Element 13 with one or more of Elements 9-12 and 14; Element 14 with one or more of Elements 9-13; Element 15 with one or both of Elements 16 and 17; Element 19 with one or more of Elements 18 and 20.

In particular, the following is a non-exhaustive list of embodiments which may or may not be claimed:
1. A surgical system, comprising:
   a surgical instrument, comprising:
   a shaft;
   an electrode arranged at an end of the shaft and energizable to provide energy to tissue;
   a conductor extending within the shaft and electrically coupled to the electrode; and
   a shield capacitively coupled to the conductor; and
   a controller in operable communication with the surgical instrument and an energy generator, wherein the controller is operable to:
      receive an input indicative of an operating mode of the surgical instrument;
      provide a voltage to the conductor and the electrode from the energy generator in the operating mode;
      determine an expected shield current based on the operating mode of the surgical instrument and the voltage;
      receive an actual shield current from the shield based on providing the voltage; and
      determine a condition of the surgical instrument based on the expected shield current and the actual shield current.
2. The surgical system of Embodiment 1, wherein determining the condition of the surgical instrument comprises comparing the actual shield current to the expected shield current.
3. The surgical system of Embodiment 2, wherein the condition of the surgical instrument comprises a low impedance condition based on the actual shield current being a threshold amount greater than the expected shield current.
4. The surgical system of Embodiment 2, wherein the condition of the surgical instrument comprises a high impedance condition based on the actual shield current being a threshold amount less than the expected shield current.
5. The surgical system of Embodiment 1, wherein the condition of the surgical instrument comprises a condition of the shield.
6. The surgical system of Embodiment 1, wherein the shaft is comprised of a conductive material.
7. The surgical system of Embodiment 1, wherein the voltage is a second voltage and the controller is further operable to:
   provide a first voltage to the conductor and the electrode;
   determine an impedance of the tissue based on providing the first voltage; and determine the second voltage to provide to the conductor and the electrode based on the determined impedance.

Therefore, the disclosed systems and methods are well adapted to attain the ends and advantages mentioned as well as those that are inherent therein. The particular embodiments disclosed above are illustrative only, as the teachings of the present disclosure may be modified and practiced in different but equivalent manners apparent to those skilled in the art having the benefit of the teachings herein. Furthermore, no limitations are intended to the details of construction or design herein shown, other than as described in the claims below. It is therefore evident that the particular illustrative embodiments disclosed above may be altered, combined, or modified and all such variations are considered within the scope of the present disclosure. The systems and methods illustratively disclosed herein may suitably be practiced in the absence of any element that is not specifically disclosed herein and/or any optional element disclosed herein. While compositions and methods are described in terms of "comprising," "containing," or "including" various components or steps, the compositions and methods can also "consist essentially of" or "consist of" the various components and steps. All numbers and ranges disclosed above may vary by some amount. Whenever a numerical range with a lower limit and an upper limit is disclosed, any number and any included range falling within the range is specifically disclosed. In particular, every range of values (of the form, "from about a to about b," or, equivalently, "from approximately a to b," or, equivalently, "from approximately a-b") disclosed herein is to be understood to set forth every number and range encompassed within the broader range of values. Also, the terms in the claims have their plain, ordinary meaning unless otherwise explicitly and clearly defined by the patentee. Moreover, the indefinite articles "a" or "an," as used in the claims, are defined herein to mean one or more than one of the elements that it introduces. If there is any conflict in the usages of a word or term in this specification and one or more patent or other documents that may be incorporated herein by reference, the definitions that are consistent with this specification should be adopted.

As used herein, the phrase "at least one of" preceding a series of items, with the terms "and" or "or" to separate any of the items, modifies the list as a whole, rather than each member of the list (i.e., each item). The phrase "at least one of" allows a meaning that includes at least one of any one of the items, and/or at least one of any combination of the items, and/or at least one of each of the items. By way of example, the phrases "at least one of A, B, and C" or "at least one of A, B, or C" each refer to only A, only B, or only C; any combination of A, B, and C; and/or at least one of each of A, B, and C.

The use of directional terms such as above, below, upper, lower, upward, downward, left, right, and the like are used in relation to the illustrative embodiments as they are depicted in the figures, the upward direction being toward the top of the corresponding figure and the downward direction being toward the bottom of the corresponding figure.

## Claims

1. A surgical system, comprising:
a surgical instrument, comprising:
a shaft;
a conductor extending within the shaft; and
a shield capacitively coupled to the conductor; and
a controller in operable communication with the surgical instrument and an energy generator, wherein the controller is operable to:
provide a voltage to the conductor from the energy generator;
receive a shield current from the shield based on providing the voltage to the conductor; and
determine a status of the surgical instrument based on the received shield current.

2. The surgical system of Claim 1, wherein the controller is further operable to determine an expected shield current based on the provided voltage.

3. The surgical system of Claim 2, wherein the controller is further operable to receive an input indicative of a selected operating mode of the surgical instrument, and wherein the expected shield current is further based on the selected operating mode.

4. The surgical system of Claim 2, wherein the controller is further operable to compare the shield current to the expected shield current and determine the status of the surgical instrument based on the comparison.

5. The surgical system of Claim 4, wherein the status of the surgical instrument comprises a low impedance condition based on the received shield current being a threshold amount greater than the expected shield current.

6. The surgical system of Claim 4, wherein the status of the surgical instrument comprises a high impedance condition based on the received shield current being a threshold amount less than the expected shield current.

7. The surgical system of Claim 1, wherein the status of the surgical instrument comprises a status of the shield.

8. The surgical system of Claim 1, wherein the shaft is comprised of a conductive material.

9. The surgical system of Claim 1, wherein the voltage is a second voltage and the surgical instrument further comprises an electrode electrically coupled to the conductor and operable to provide energy to tissue, wherein the controller is further operable to:
provide a first voltage to the tissue from the energy generator via the conductor and the electrode;
determine an impedance of the tissue based on providing the first voltage; and
determine the second voltage to provide to the conductor based on the determined impedance.

10. A non-transitory computer readable medium storing instructions that, when executed by a processor, cause the processor to:
receive an input indicative of an operating mode of a surgical instrument;
control an energy generator to provide a voltage to a conductor of the surgical instrument, the conductor being capacitively coupled to a shield of the surgical instrument;
determine an expected shield current based on the operating mode of the surgical instrument and the voltage;
receive an actual shield current from the shield based on providing the voltage;
compare the expected shield current and the actual shield current; and
detect an abnormality in the surgical instrument based on the comparison.

11. The non-transitory computer readable medium of Claim 10, wherein the abnormality comprises a high impedance condition of the surgical instrument based on the actual shield current being a threshold amount below the expected shield current.

12. The non-transitory computer readable medium of Claim 10, wherein the abnormality comprises a low impedance condition of the surgical instrument based on the actual shield current being a threshold amount above the expected shield current.

13. The non-transitory computer readable medium of Claim 10, wherein the abnormality of the surgical instrument comprises an abnormality in the shield.
